# EUROPEAN PATENT SPECIFICATION

(11) **EP 0 676 198 B1**
(45) Date of publication and mention of the grant of the patent: **07.10.1998**
(21) Application number: 95301981.7
(22) Date of filing: 24.03.1995
(51) Int. Cl.: A61K 31/415, A61K 31/57

(54) **Fungicidal compositions containing a combination of bifonazole and fluocinonide**
Fungizide Zusammensetzungen enthaltend eine Kombination von Bifonazol und Fluocinonid
Compositions fongicides contenant une combinaison de bifonazole et de fluocinonide

(30) Priority: 05.04.1994 IL 10923094
(43) Date of publication of application: 11.10.1995
(73) Proprietor: AGIS INDUSTRIES (1983) LTD, Bnei-Brak 51200 (IL)
(72) Inventor: Zighelboim, Marcel, Kiriat Motzkin 26381 (IL); Sintov, Amnon, Omer 84965 (IL)
(74) Representative: Evans, David Charles

(56) References cited:
- MYCOSES, vol. 32,no. 3, 1989 pages 139-144, GILLIAN S. SHANKLAND ET AL. 'Treatment of experimental dermatophytosis in guinea pigs: A comparative study of bifonazole and a bifonazole-hydrocortisone combination'
- DRUGS, vol. 36,no. sup5, 1988 pages 9-14, B.GIANNOTTI 'Current treatment guidelines for topical corticosteroids'
- CUTIS, vol. 30, 1982 pages 258-261, MARTIN H. WORTZEL 'A double blind study comparing the superiority of a combination antifungal (clotrimazole)/steroidal (betamethasone dipropionate) product'
- PAEDIATRIC CLINICS OF NORTH AMERICA, vol.30, no., 1988, page 749 - 765, ''

## Description

The present invention relates to a novel topical antifungal delivery system. More particularly the present invention relates to a new and useful topical pharmaceutical composition, comprising a combination of anti-fungal/anti-inflammatory drugs for more efficacious treatment of diseased skin and mucosa.

The usefulness of corticosteroid and anti-mycotic combinations in the treatment of dermatoses associated with fungal infections and similar conditions has long been recognized by dermatologists. It has also been demonstrated in clinical studies that the co-administration of a fungicidal medication and a topical steroid speeds the clinical relief and antifungal improvement (MH Wortzel, Cutis 30:258-261 1982; M McKay, Am. J. Obstet. Gynecol. 158:991-993 1988). Currently, there are three formulations combining a corticosteroid and an anti-fungal agent in the local drug market. These are Daktacort^{(R)}, containing 2% miconazole nitrate and 1% hydrocortisone, to be applied topically two or three times daily; Travocort^{(R)}, containing 1% isoconozole nitrate and 0.1% diflucortolone valerate, to be applied twice daily; and Hydroagisten^{(R)}, containing 1% clotrimazole and 1% hydrocortisone acetate.

It has now been suprisingly found that a combination of bifonazole and fluocinonide can be used to produce a pharmaceutical composition for application to the skin and/or mucosal tissue for treating a dermatological disease or condition and which composition when applied once daily is at least as effective as the prior art combinations, which are applied twice daily.

Mycoses vol 32, no. 3, 1989, p. 139-144 describes compositions containing bifonazole and hydrocortisone. Cutis, vol. 30, 1982, pages 258-261 describes compositions containing clotrimazole and betamethasone.

Thus, the present invention provides a topical antifungal/ corticosteroid composition which comprises bifonazole as a fungicide and fluocinonide as an anti-inflammatory steroid; in a pharmaceutically acceptable vehicle.

Bifonazole is a substituted imidazole derivative which is structurally related to other drugs, such as clotrimazole, econazole, miconazole, sulconazole and oxiconazole. Bifonazole inhibits the growth of a broad range of dermatophytes (e.g., Trichophyton, Microsporum), yeasts (e.g., Candida spp., Pityrosporum, Torulopsis glabrata), moulds (e.g., Aspergillus spp.) and various fungi (e.g., Coccidioides, Blastomyces, Histoplasma). It is also active against some Gram-positive bacteria (e.g., Staphylococci, Streptococci, etc.). Bifonazole is presumed to inhibit ergosterol synthesis leading to functional irregularities in the microorganism's cell membrane and ultimately destroying its ability to maintain the intracellular medium. Some of the superficial mycoses that are commonly treated by bifonazole topical preparations are tinea cruris, tinea pedis, tinea manuum, candidiasis, pityriasis versicolor, and onychomycosis. Also, an improvement was found in patients suffering of Rosacea, Psoriasis, Sebopsoriasis and Seborrhoeic dermatitis (HF Doering, Dermatologica 169(supp 1):125-134, 1984; GP Ford et al., ibid 169(supp 1):135-140, 1984).
Fluocinonide (fluocinolone acetonide 21-acetate) is a corticosteroid used topically in the treatment of various skin disorders.

Despite its availability and use, Bifonazole has never been available in combination with a corticosteroid drug to provide combined anti-inflammatory activity and rapid relief from symptoms of fungal infections, and nowhere has it been suggested that by combining and mixing effective amounts of bifonazole and fluocinonide in a pharmaceutical base, a high degree of efficacy and tolerability is achieved when the composition is administered in only a once daily regimen.

Thus, in its preferred embodiments, the present invention provides novel compositions, together with instructions for once-daily administration, while other commercial products that contain such combinations are recommended as being effective in twice-or thrice-daily application. It is well known that patient compliance with the treatment is negatively correlated with the frequency of the administration; therefore, a once daily dosage form provides higher compliance for patients and consequently higher clinical success rates. Unfortunately, a vast majority of preparations in the market are not indicated for a once-daily regimen. Furthermore, in the U.S. and Israel there is not even a single antifungal/corticosteroid combination given once a day.

Thus, according to the present invention there is now provided for the first time an antifungal topical composition for once daily use which comprises a steroidal anti-inflammatory drug combinations. More specifically, the invention provides a system containing between about 0.5 and about 2% of bifonazole and between 0.02 and 0.1% of fluocinonide in topical pharmaceutically acceptable vehicles The pharmaceutically acceptable vehicle is preferably selected from the group consisting of an oil-in-water or water-in-oil emulsion, solution, suspension, gel, aerosol, or powder. Oil-in-water or water-in-oil emulsions are formulated in ways that a stable topical ointment, lotion, cream, stick, or foam is achieved. The stabilization of the topical emulsions may be established and optimized by using the preferred combinations of hydrophilic and lipophilic emulsifiers properly-aligned at the water/oil interface. The emulsifying agents and their concentrations and proportions may be chosen according to the principle of the well-established H.L.B. method published by W.C. Griffen (H.L.B. - the hydropilic- lipophilic balance, J. Soc. Cos. Met. Chem. 5, 249, 1954). In the case where the composition according to the invention is an emulsion, the oil phase is selected from the group consisting of bees-wax, spermaceti, 2-octyl dodecanol, lanolin, sodium C12-15 alcohols sulphate, esters of fatty acids and high-molecular weight alcohols such as cetyl palmitate and cetearyl octanoate, esters of fatty acids and branched alcohols or polyols such as isopropyl palmitate or myristate, cocoglycerides, cosbiol, wool alcohols, cocoa butter, stearyl alcohol, cholesterol, liquid paraffin, soft paraffin, hard paraffin or the like.

The emulsifying agents used for the purpose of dispersion of above-mentioned fats or oils and the like in the aqueous phase are advantageously selected from the group of non-ionic surfactants consisting of sorbitan sesquioleate, PEG-5 glyceryl stearate, poloxamers, cetostearyl alcohol, polysorbate 60, sorbitan monostearate, sorbitan monooleate, or glyceryl monostearate. In the case where the composition according to the invention is a gel or solution, the composition comprises bifonazole, fluocinonide, a lower alkanol having from one to four carbons, water, a gelifying agent (if a gel), one or more polyhydric alcohols selected from the group consisting of a lower alkylene glycol having from two to four carbons, glycerine and polyethylene glycol having an average molecular weight from 200 to 2000, and bifonazole-solubilizing amount of an organic acid, preferably lactic acid. The gelifying agents are selected from the group consisting of polysaccharides such as cellulose derivatives, acrylic polymers, proteins, polyhydroxy compounds such as polyethylene glycol having an average molecular weight from 400 to 2000, and polyoxyethylene-3-cetylstearyl alcohol, known as Emulgin B3.

All semi-solid topical pharmaceutical preparations should preferably be stable and consistent, non-leaky, non-staining, and non-greasy. In the case where the composition according to the invention is a powder, the composition comprises bifonazole, fluocinonide, and a diluting powder compound suitable as a lubricant. This lubricant is selected from the group consisting of talc, microcrystalline cellulose, polyvinyl pyrrolidone, metal stearates, lactose or starch known to have non-irritating. non-toxic and inert properties.

The bifonazole/fluocinonide combination could be topically applied in a slow-release manner using an adhesive sponge bandage, or alternatively a gauze or sponge sandwich containing a layer of the active principals of the invention situated between an upper and lower absorbent layer.

The bifonazole/fluocinonide combination of this invention can also be applied onto the mucosa, for example as a buccal gel or vaginal preparation. For this purpose, several bioadhesive polymers are selected from the group consisting of polyethylene glycols, cellulose derivatives, starch, and polyacrylic acid such as polycarbophil and Carbopol 934.

In accordance with the present invention, the vehicle, can be altered to allow for its use on diverse skin areas or conditions, and on different types of mucous membranes. Thus, as described herein, the vehicles can be in the form of a cream, lotion, ointment, gel, stick, topical solution, foam, spray, shampoo, or powder. From the point of view regarding the formulation characteristics, the pharmaceutical preparations could be processed as an water-in-oil or oil-in-water emulsion, clear solution, gel solution, aerosol, powder mix, film-forming liquid, bioadhesive preparation, detergents- containing gel, suspension in gel, liquid, or emulsion, etc.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example 1

### Water-in oil ointment

| Ingredient | Percent by weight |
|---|---|
| 1) Bifonazole | 1.0 |
| 2) Fluocinonide | 0.05 |
| 3) DEHYMULS® E | 7.0 |
| 4) White bees-wax | 4.0 |
| 5) White vaseline | 35.0 |
| 6) Heavy liquid Paraffin | 23.9 |
| 7) Purified water | q.s. |
| DEHYMULS® E = sorbitan sesquioleate (and) penta-erythrityl tetracocoate (and) stearyl citrate (and) bees-wax (and) aluminum stearate. | |

Emulsification is taken place by heating the oil phase (ingredients 3-6) to 88^{o}C, and blending while mixing into the water, preheated to 75^{o}C. While mixing, the active ingredients are gradually added at 70^{o}C.

### Example 2

### Oil-in-water cream

| Ingredient | Percent by weight |
|---|---|
| 1) Bifonazole | 1.00 |
| 2) Fluocinonide | 0.05 |
| 3) ARLATONE® 983 S | 6.25 |
| 4) CUTINA® CBS | 8.75 |
| 5) Cetearyl octanoate | 3.75 |
| 6) Propylene glycol | 15.60 |
| 7) Glycerine (85%) | 1.90 |
| 8) Benzoic acid | 0.25 |
| 9) Purified water | q.s. |
| ARLATONE® 983 S = polyoxyethylene-5-glyceryl stearate CUTINA® CBS = glyceryl stearate (and) cetearyl alcohol (and) cetyl palmitate (and) coco-glycerides | |

The oil phase (ingredients 3-5) is heated to 80^{o}C while mixing until a uniform liquid is achieved. Then, ingredients 6-8 are added into the hot liquid and thoroughly mixed. In a separate vessel, fluocinonide is dissolved in 80^{o}C- preheated water. The oil phase is then added into the aqueous phase while mixing and homogenizing at 80^{o}C. After cooling to 70^{o}C, bifonazole is slowly added while homogenizing at high shear.

### Example 3

### Oil-in-water cream

| Ingredient | Percent by weight |
|---|---|
| 1) Bifonazole | 1.0 |
| 2) Fluocinonide | 0.05 |
| 3) ARLACEL® 60 | 3.0 |
| 4) TWEEN® 60 | 1.5 |
| 5) Walrat (spermaceti) | 5.0 |
| 6) LANNETE® O | 12.0 |
| 7) EUTANOL® G | 10.0 |
| 8) Benzyl alcohol | 1.0 |
| 9) Purified water | q.s. |
| ARLACEL® 60 = sorbitan monostearate TWEEN® 60 = polyoxyethylene sorbitan monostearate LANNETE® O = cetearyl alcohol EUTANOL® G = 2-octyl dodecanol | |

The oil phase (ingredients 3,5,6,7,8) is heated while stirring to 80^{o}C. Fluocinonide is added then to the oil phase and uniformly dispersed. The oil phase is emulsified into part of the water (90%) preheated to 80^{o}C. Tween 60 is dissolved into the remaining water, and bifonazole is then suspended. The suspension is added to the emulsion and the mixing and blending continued until the whole preparation is cooled.

### Example 4

### Oil-in-water cream

| Ingredient | Percent by weight |
|---|---|
| 1) Bifonazole | 1.0 |
| 2) Fluocinonide | 0.05 |
| 3) ARLACEL® 60 | 1.0 |
| 4) TWEEN® 60 | 3.5 |
| 5) Vaseline | 10.0 |
| 6) LANNETE® O | 5.0 |
| 7) Heavy liquid paraffin | 10.0 |
| 8) Purified water | q.s. |
| ARLACEL® 60 = sorbitan monostearate TWEEN® 60 = polyoxyethylene sorbitan monostearate LANNETE® O = cetearyl alcohol | |

Ingredients 3,4,6,7,8 are heated at 80^{o}C until uniform liquid is obtained. Perform emulsification at 80^{o}C in part of the water (about 90%). In a separate vessel, dissolve ingredient 5 in the remaining water. Suspend bifonazole and fluocinonide in the detergent-containing solution. Add the suspension into the emulsion at 70^{o}C.

### Example 5

### Water-in-oil Ointment

| Ingredient | Percent by weight |
|---|---|
| 1) Bifonazole | 1.0 |
| 2) Fluocinonide | 0.05 |
| 3) Vaseline | 50.0 |
| 4) LANNETE® WAX SX | 7.0 |
| 5) Lanolin | 14.0 |
| 6) Purified water | q.s. |
| LANNETE WAX SX = cetearyl alcohol (and) sodium C12-15 alcohols sulphate | |

Bifonazole and fluocinonide are added into a 60^{o}C preheated vaseline and mixed until a uniform dispersion is obtained. Ingredient 4 is heated to 80^{o}C and added to the dispersion. Water is then heated to 60^{o}C and added into the oil phase.

### Example 6

### Gel Preparation

| Ingredient | Percent by weight |
|---|---|
| 1) Bifonazole | 1.0 |
| 2) Fluocinonide | 0.05 |
| 3) EMULGIN® B3 | 20.0 |
| 4) CETIOL® HE | 20.0 |
| 5) Isopropyl isostearate | 5.0 |
| 6) Ethyl alcohol | 3.0 |
| 7) Lactic acid | 1.5 |
| 8) Benzyl alcohol | 1.0 |
| 9) Purified water | q.s. |
| EMULGIN® B3 = polyoxyethylene-30 cetylstearyl alcohol CETIOL® HE = polyehylene glycol 7 glyceryl cocoate | |

Ingredients 3, 4, 5, and 8 were heated to 80^{o}C while mixing, then bifonazole and fluocinonide were added and completely dissolved. In a separate vessel, lactic acid is dissolved in 80oC preheated water (about 80% of total). The lactic acid solution is then added to the former solution while mixing and blending. At 60^{o}C, the remaining water and the ethyl alcohol are added and the whole gel is then cooled.

### Example 7

### Shampoo Preparation for Scalp treatment

| Ingredient | Percent by weight |
|---|---|
| 1) Bifonazole | 1.0 |
| 2) Fluocinonide | 0.05 |
| 3) ZOHARPON® LAS 30 | 33.8 |
| 4) ETA® 27 | 24.0 |
| 5) REWOMINOX® B-204 | 2.5 |
| 6) LAURAMIDE®-R | 2.5 |
| 7) Ethyl alcohol | 3.0 |
| 8) Lactic acid | 0.8 |
| 9) Fragnance | 0.4 |
| 10) Purified water | q.s. |
| ZOHARPON® LAS 30 = sodium lauryl sulfate (30% sol.) ETA® 27 = sodium laureth sulfate (27% sol.) REWOMINOX® B-204 = cocamidopropylamine oxide LAURAMIDE®-R = cocamide diethylamine (90% sol.) | |

Ingredients 3, 4, and 9 are dissolved in water at room temperature. In a separate vessel, bifonazole and fluocinonide, and lactic acid are dissolved in the ethyl alcohol. The two solution are then combined. Ingredients 5 and 6 are added to the solution and mixed until a clear solution is formed.

### Example 8

### Foam Preparation

| Ingredient | Percent by weight |
|---|---|
| 1) Bifonazole | 1.0 |
| 2) Fluocinonide | 0.05 |
| 3) CETIOL® HE | 9.0 |
| 4) Triethanolamine | 0.6 |
| 5) TRANSCUTOL® | 17.0 |
| 6) Ethyl alcohol | 16.7 |
| 7) Preservatives | 0.04 |
| 8) BRIJ® 35 | 0.6 |
| 9) ARLACEL® 186 | 0.16 |
| 10) Purified water | q.s. |
| propane/butane/isobutane 19:2:77 + 5.0% CETIOL® HE = polyehylene glycol 7 glyceryl cocoate TRANSCUTOL® = diethylene glycol monoethyl ether BRIJ® 35 = polyoxyethylene 23 lauryl alcohol ARLACEL® 186 = glyceryl mono and dioleate | |

Bifonazole, fluocinonide and the preservatives (parabens) are dissolved in a mixture of transcutol, triethanolamine and ethyl alcohol at 35^{o}C. In a separate vessel, Brij® 35, Arlacel® 186 and Cetiol® HE are dissolved in water at ambient temperature. The two solutions are eventually combined and filled in an aerosol container and sealed. The propellant gas is then filled under pressure.

### Example 9

### Topical solution

| Ingredient | Percent by weight |
|---|---|
| 1) Bifonazole | 1.0 |
| 2) Fluocinonide | 0.05 |
| 3) Polyethylene glycol 400 | q.s. |

Bifonazole and fluocinonide are dissolved in PEG 400 at 70^{o}C while stirring.

### Comparative Example 10

### Effectiveness of two imidazole-corticosteroid Formulations - A Double-Blind Clinical Trial

The bifonazole-fluocinonide combination of the present invention, and in particularly, the formulation of example 3 was compared with a commercial product containing 2% miconazole and 1% hydrocortisone (Daktacort^{(R)}, Abic). The present formulation combining 1% bifonazole with 0.05% fluocinonide, to be applied once daily, was compared in a double blind trial against the established formulation - Daktacort^{(R)} as follows:

### Patients and Methods:

Thirty patients, 22 men and 8 women, age 14 to 73 years, were enrolled in the study. Informed consent was signed by all patients or their parents. The candidates were patients suffering from bilateral, almost symmetrical fungal infections, mostly tinea cruris (table 1). Samples from all the patients were taken and a positive KOH test was a prerequisite for inclusion in the study. Further cultures were done from all the samples. Exclusion criteria were age younger than 14 years, pregnancy and previous use of any anti-fungal drug, whether systemic or topical. The patients were requested not to participate in another clinical trial throughout the study period. Treatment assignment was double blind and randomized between the two sides of the skin lesion to be treated. Each patient received 2 pairs of identical tubes. One pair contained miconazole-hydrocortisone, with either morning/evening label, the other pair of tubes was placebo cream (morning), and the bifonazole-fluocinonide combination (evening). Right and left sides were assigned at random. Patients were instructed to apply the creams twice daily for at least 10 days. Clinical examination at entrance to the study evaluated the patients in relation to 5 signs of the fungal infection: redness, scaling, edema and vesiculation, oozing and scratch marks. Further evaluation was planned on the follow-up visit 11-15 days later, with a 3-point scale of response: good, moderate, and no-change. Repeated KOH test was done in the follow-up visit on both sides. A questionnaire related to the symptoms of infection, the day and the side of initial improvement was marked by the patients.

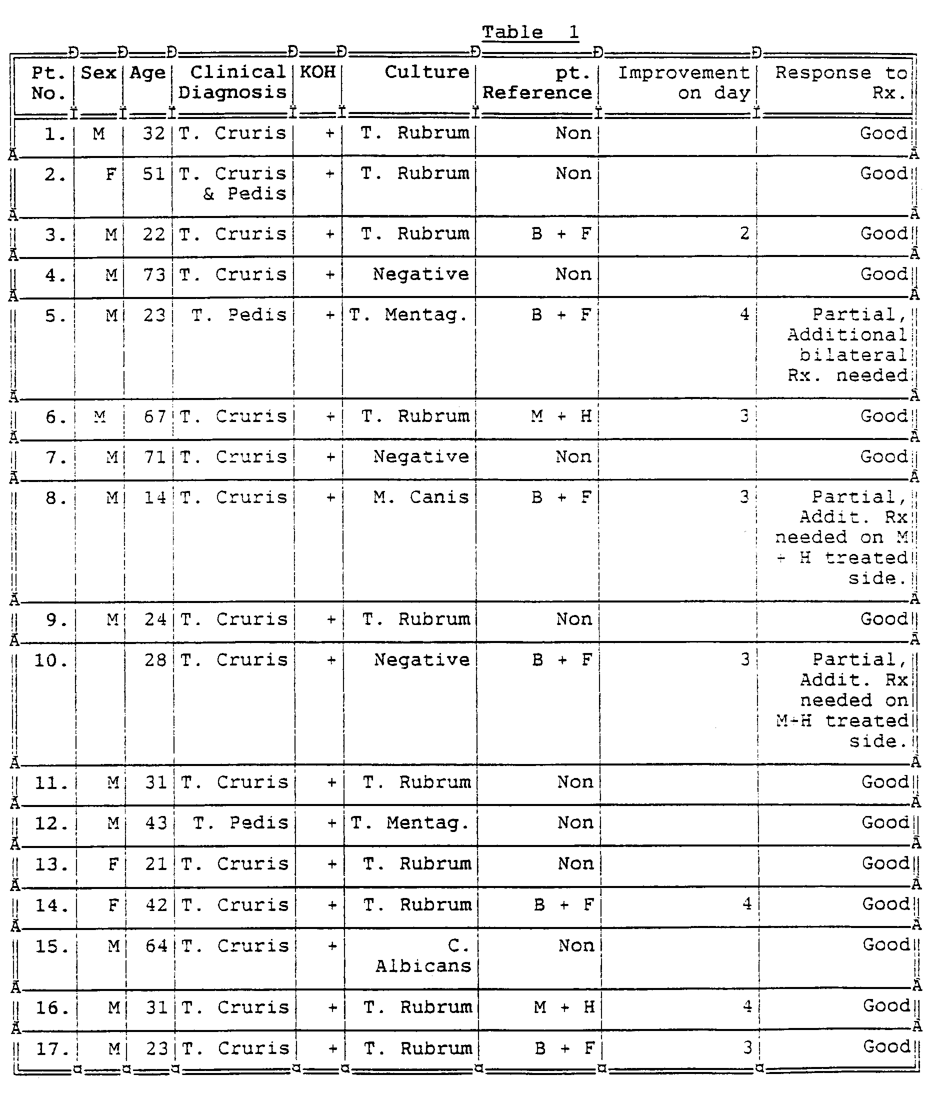

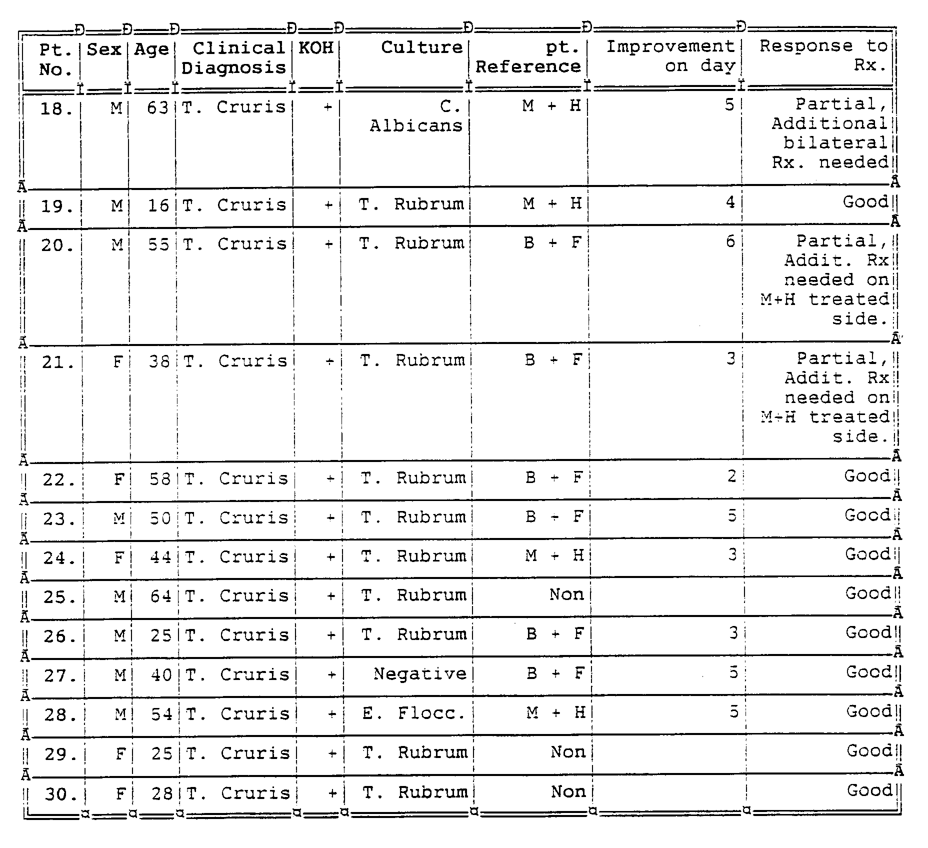

### Results (table 1):

Mycologic confirmation by culture was obtained in 26 of the 30 patients. Trichophyton rubrum was isolated from 20 patients, T. mentagrophytes from two, Candida sibicans from two, while E. floccosum and M. canis grew in one culture each. Clinical response was mostly satisfactory, and no side effects were observed. Out of the 30 study patients, twelve were equally satisfied from both formulations, twelve marked the side treated by bifonazole-fluocinonide as the one improved first, while the other six reported the opposite. The first improvement was felt by the patients preferring bifonazole-fluocinonide between the second to sixth day of treatment (mean 3.6), while those patients preferring the miconazole-hydrocortisone combination mentioned day 3-5 with day 4 as the average. Clinical examination at the follow-up visit confirmed the patients reports, although bilateral improvement was already noticed in most of them, except those requiring additional treatment. The repeated KOH test following treatment was negative on both sides in all the patients. Twenty-four of the patients showed good response, while six patients with moderate response required further treatment, two of them on both sides and the other 4 on the side treated by the miconazole-hydrocortisone formula. These results are summarized in the following table:

| SIDE TREATED WITH M + H | | | | |
|---|---|---|---|---|
| | | Success | Failure | Total |
| | Success | 24 | 4 | 28 |
| Side treated with B = F | | | | |
| | Failure | 0 | 2 | 2 |
| | Total | 24 | 6 | 30 |

This table exhibits overwhelming supporting evidence for the model stating that the two drugs are equally likely to succeed/fail in treating patients as is measured by the proportion on the diagonal (26/30 = 86.7%). The relevant statistical test (McNemmar test distributed as chi-square with 1 degree of freedom) given a p value under .005 (5 in thousand).

The somewhat better performance of Bifonazole + fluocinonide as indicated by more treatment failure in the miconazole + hydrocortizone, compared to Bifonazole + fluocinonide is also reflected in the patient questionnaire, whereby more patients have rated the Bifonazole + fluocinonide as more effective than the other treatment.

### Conclusions:

Both bifonazole + fluocinonide and miconazole + hydrocortizone were found to be effective in the treatment of fungal infections of the skin (p < 0.005).Twice as many patients preferred the treatment of bifonazole + fluocinonide. While bifonazole + fluocinonide cream is at least as effective as miconazole + hydrocortizone cream, it has the advantage of being a "once a day" treatment with a chance for better patient compliance.

## Claims

1. A topical antifungal/corticosteroid composition which comprises:
(a) bifonazole as a fungicide; and
(b) fluocinonide as an anti-inflammatory steroid; in a pharmaceutically acceptable vehicle.

2. A composition as claimed in claim 1, comprising 0.5%-2% bifonazole and 0.02%-0.1% fluocinonide.

3. A composition as claimed in claim 1, wherein the pharmaceutically acceptable vehicle is selected from the group consisting of oil-in-water and water-in-oil emulsions.

4. A composition as claimed in claim 1, wherein the pharmaceutically acceptable vehicle is an emulsion which is provided in the form of a cream, lotion, ointment, stick, foam, spray or shampoo.

5. A composition as claimed in claim 1, wherein the pharmaceutically acceptable vehicle is selected from the group consisting of gelified solutions, suspensions, foams, and emulsions.

6. A composition as claimed in claim 1, wherein the pharmaceutically acceptable vehicle is selected from the group consisting of solutions, suspensions and polymeric liquids.

7. A composition as claimed in claim 1, wherein the pharmaceutically acceptable vehicle is a lubricating powder.

8. A composition as claimed in claim 1, wherein the pharmaceutically acceptable vehicle has bioadhesive properties.

9. A composition as claimed in claim 1, wherein the pharmaceutically acceptable vehicle is an adhesive bandage having absorbant layers.

10. A composition according to claim 1 in combination with instructions for once daily application.

11. The use of the ingredients of a composition as claimed in claim 1 in the preparation of a pharmaceutical agent for application to the skin and/or mucosal tissue of a human or animal, for treating a dermatological disease or condition.

## Patentansprüche

1. Auftragbare antimykotische/corticosteroide Zusammensetzung mit:
(a) Bifonazol als ein Fungizid; und
(b) Fluocinonid als ein entzündungshemmendes Steroid; in einem Arzneistoffträger.

2. Zusammensetzung nach Anspruch 1, die 0,5%-2% Bifonazol und 0,02%-0,1% Fluocinonid aufweist.

3. Zusammensetzung nach Anspruch 1, wobei der Arzneistoffträger aus der Gruppe, die aus "Öl-in-Wasser"-und "Wasser-in-Öl"-Emulsionen besteht, gewählt wird.

4. Zusammensetzung nach Anspruch 1, wobei der Arzneistoffträger eine Emulsion darstellt, die in Form einer Creme, Lotion, Salbe, eines (Fett)-Stifts, Schaums, Sprays oder Shampoos zur Verfügung gestellt wird.

5. Zusammensetzung nach Anspruch 1, wobei der Arzneistoffträger aus der Gruppe, die aus gelartigen Lösungen, Suspensionen, Schäumen und Emulsionen besteht, gewählt wird.

6. Zusammensetzung nach Anspruch 1, wobei der Arzneistoffträger aus der Gruppe, die aus Lösungen, Suspensionen und polymeren Flüssigkeiten besteht, gewählt wird.

7. Zusammensetzung nach Anspruch 1, wobei der Arzneistoffträger einen fettenden Puder darstellt.

8. Zusammensetzung nach Anspruch 1, wobei der Arzneistoffträger bioadhäsive Eigenschaften besitzt.

9. Zusammensetzung nach Anspruch 1, wobei der Arzneistoffträger einen Klebeverband darstellt, der absorbierende Schichten besitzt.

10. Zusammensetzung nach Anspruch 1 verbunden mit Anweisungen für einmal tägliche Anwendung.

11. Verwendung der Bestandteile einer Zusammensetzung nach Anspruch 1 für die Herstellung eines pharmazeutischen Mittels zum Auftragen auf die Haut und/oder Schleimhaut eines Menschens oder eines Tieres, zur Behandlung einer dermatologischen Krankheit oder Beschwerde.

## Revendications

1. Une composition antifongique/corticostéroïde topique, qui comporte :
a) du bifonazole comme fongicide ; et
b) du fluocinonide comme stéroïde anti-inflammatoire ; dans un véhicule pharmaceutiquement acceptable.

2. Une composition telle que revendiquée dans la revendication 1, comportant 0,5 % - 2 % de bifonazole et 0,02 % - 0,1 % de fluocinonide.

3. Une composition telle que revendiquée dans la revendication 1, dans laquelle le véhicule pharmaceutiquement acceptable est choisi dans le groupe consistant en des émulsions huile dans eau et eau dans huile.

4. Une composition telle que revendiquée dans la revendication 1, dans laquelle le véhicule pharmaceutiquement acceptable est une émulsion qui est fournie sous la forme de crème, de lotion, d'onguent, de bâton, de mousse, de pulvérisation ou de shampoing.

5. Une composition telle que revendiquée dans la revendication 1, dans laquelle le véhicule pharmaceutiquement acceptable est choisi dans le groupe consistant en des solutions gélifiées, des suspensions, des mousses et des émulsions.

6. Une composition telle que revendiquée dans la revendication 1, dans laquelle le véhicule pharmaceutiquement acceptable est choisi dans le groupe consistant en des solutions, des suspensions et des liquides polymères.

7. Une composition telle que revendiquée dans la revendication 1, dans laquelle le véhicule pharmaceutiquement acceptable est une poudre lubrifiante.

8. Une composition telle que revendiquée dans la revendication 1, dans laquelle le véhicule pharmaceutiquement acceptable présente des propriétés bioadhésives.

9. Une composition telle que revendiquée dans la revendication 1, dans laquelle le véhicule pharmaceutiquement acceptable est un bandage adhésif comportant des couches absorbantes.

10. Une composition selon la revendication 1, en combinaison avec des instructions pour une application une fois par jour.

11. L'utilisation des ingrédients d'une composition telle que revendiquée dans la revendication 1 dans la préparation d'un agent pharmaceutique pour l'application à la peau et/ou au tissu de muqueuse de l'homme ou de l'animal, en vue du traitement d'une maladie ou d'une condition dermatologique.
